# FASCICULE DE BREVET EUROPEEN

(11) **EP 1 037 522 B1**
(45) Date de publication et mention de la délivrance du brevet: **02.07.2003**
(21) Numéro de dépôt: 98959944.4
(22) Date de dépôt: 10.12.1998
(51) Int. Cl.: A01H 1/06, A01H 5/10

(54) **OBTENTION DE MUTANTS CLEISTOGAMES DE CRUCIFERES**
HERSTELLUNG VON CLEISTOGAMEN MUTANTEN VON KIENZBLÜTLERN
METHOD FOR OBTAINING CLEISTOGAMOUS MUTANTS OF CRUCIFERS

(30) Priorité: 12.12.1997 FR 9715768
(43) Date de publication de la demande: 27.09.2000
(73) Titulaire: INSTITUT NATIONAL DE LA RECHERCHE AGRONOMIQUE (INRA), 75341 Paris Cédéx 07 (FR)
(72) Inventeur: RENARD, Michel, F-35650 Le Rheu (FR); TANGUY, Xavier, F-35650 Le Rheu (FR)
(74) Mandataire: Vialle-Presles, Marie José
(86) Numéro de dépôt international: FR9802681
(87) Numéro de publication internationale: WO99030555

(56) Documents cités:
- DATABASE WPI Section PQ, Week 9408 Derwent Publications Ltd., London, GB; Class P13, AN 94-063456 XP002076327 & SU 1 789 138 A (AS TADZ COTTON GEN GENETICS SECT)

## Description

L'invention est relative à l'obtention de mutants cléistogames de crucifères, et en particulier du colza, et à leurs utilisations.

Le colza (*Brassica napus L*.) est une crucifère, issue de l'hybridation spontanée entre le chou (*Brassica rapa*) et la navette (*Brassica oleracea*). Il s'agit d'une espèce à reproduction mixte (allogame ou autogame) pour laquelle le taux de fécondation croisée est d'environ 30%.

Les variétés de colza cultivées jusqu'à maintenant présentent des fleurs à 4 pétales qui s'épanouissent à l'anthèse. La coupe et le diagramme d'une fleur de colza sont représentés par les Figures lA et 1B : (1) pétale ; (2) sépale ; (2') sépale bossu ; (3) nectaire ; (4) étamine courte ; (5) étamine longue ; (6) stigmate ; (7) pistil ; (8) ovaire ; (9) pédoncule.

Le pollen de colza peut être transporté par le vent, ou les insectes pollinisateurs (en particulier les abeilles qui visitent la fleur de colza en position surmontante favorable à la pollinisation) sur des distances de plusieurs kilomètres.

Ceci occasionne des difficultés lorsque l'on souhaite éviter une fécondation croisée entre différentes variétés de colza, par exemple lors de la sélection de lignées pures, ou de la production de semences. En outre, le colza est capable de s'hybrider avec des crucifères sauvages, telles que la ravenelle, ce qui pose un problème supplémentaire lors de l'expérimentation en champ de colzas génétiquement modifiés.

Ceci oblige à poser des sachets d'autofécondation sur les hampes florales, pour la sélection et la multiplication de lignées pures, à isoler la parcelle de multiplication d'au moins 400 m d'autres cultures de colza, pour la production de semences. Dans le cas de colzas génétiquement modifiés, il faut en outre, afin de limiter les risques de flux de pollen, entourer la parcelle par une zone de culture de colza non-modifié de 6 mètres de large, dénommée « bande tampon », qui joue le rôle de « piège à pollen » ; il faut également à s'assurer de l'absence, dans le périmètre entourant l'essai, de crucifères sauvages capables de s'hybrider spontanément avec le colza.

Il apparaît donc que les techniques actuellement utilisées pour prévenir la dissémination du pollen de colza entraînent de nombreuses contraintes ; les Inventeurs ont recherché comment les limiter.

On connaît dans diverses familles de plantes, des espèces (par exemple, l'arachide, le riz, le sorgho, le lin, la fétuque) possédant des fleurs cléistogames, c'est à dire des fleurs chez lesquelles les enveloppes florales ne s'ouvrent pas. La fleur reste close, et la fécondation s'accomplit par autopollinisation à l'intérieur de celle-ci. Chez ce type de plantes, le pollen est donc confiné dans la fleur, et aucune mesure supplémentaire n'est nécessaire pour éviter sa propagation.

Toutefois, l'utilisation de colza cléistogame n'avait jamais été envisagée auparavant ; en effet, l'existence de plantes possédant cette caractéristique n'ayant jamais été rapportée chez les crucifères, on ignorait si elle pouvait survenir dans cette famille.

Les Inventeurs ont entrepris de déterminer s'il était cependant possible d'obtenir des mutants de colza cléistogames.

Dans ce but, ils ont procédé à la mutagénèse artificielle de graines de colza, et ont observé, dans la descendance des plantes issues des graines mutées, l'existence de mutants cléistogames. Ils ont en outre constaté que chez ces mutants, la cléistogamie était compatible avec un développement et une reproduction normale de la plante, et ont montré que ce caractère était donc contrôlé par un seul gène, dénommé ci-après "*clg1*".

La présente invention a pour objet un procédé d'obtention d'un mutant cléistogame de crucifère, caractérisé en ce qu'il comprend au moins les étapes suivantes :
a) la mutation dans au moins une cellule de crucifère, d'au moins une copie du gène contrôlant la cléistogamie, dénommé "*clgl*" ;
b) l'obtention, à partir de ladite cellule, d'une plante adulte présentant à maturité florale des fleurs totalement closes.
   Selon un mode de mise en oeuvre préféré du procédé conforme à l'invention l'étape b) comprend les sous-étapes suivantes :
c) l'obtention de plantes adultes présentant à maturité florale des fleurs incomplètement ouvertes ;
d) le croisement desdites plantes entre elles, et la sélection parmi leurs descendants, d'au moins une plante adulte présentant à maturité florale des fleurs totalement closes.

Pour la mise en oeuvre de l'étape a) du procédé conforme à l'invention, on peut avantageusement utiliser comme matériel de départ, des semences de crucifère ; on peut également utiliser toute cellule, tissu, ou organe à partir desquels il est possible de régénérer la plante entière.

La mutation du gène contrôlant la cléistogamie peut être induite par tout moyen usuel de mutagenèse. On peut par exemple traiter des semences de crucifères par un mutagène ; on peut ainsi procéder à une mutagenèse par irradiation, ou à une mutagenèse chimique en traitant les semences par un produit mutagène tel qu'un sulfonate, par exemple le di-éthyl sulfonate, ou l'éthyl-méthyl sulfonate. On peut également provoquer la mutation souhaitée en utilisant les outils habituels du génie génétique.

Le procédé conforme à l'invention peut par exemple être mis en oeuvre pour l'obtention de mutants cléistogames du colza, ainsi que du chou, de la navette ou de la moutarde, en particulier la moutarde brune (*Brassica juncea*).

L'invention a également pour objet des mutants cléistogames de crucifères, susceptibles d'être obtenus par le procédé conforme à l'invention.

Dans le cas du colza, par exemple, des mutants conformes à l'invention peuvent être utilisés dans toutes les applications où il est nécessaire d'éviter une fécondation croisée entre différentes variétés de colza, ou entre le colza et des crucifères adventices telles par exemple que la ravenelle. Il s'agit en particulier de la fixation et de multiplication de lignées pures, de la production de semences, et de la production de colza à usages industriels ou de variétés transgéniques.

L'utilisation de mutants conformes à l'invention permet en effet, dans ces différentes applications, de simplifier les contraintes d'isolement, par exemple en supprimant la nécessité d'utiliser des sachets d'autofécondation, en permettant de réduire la distance de séparation entre les différentes cultures, et, de manière générale en évitant ou en limitant l'emploi de dispositifs destinés à prévenir la propagation du pollen.

L'utilisation de mutants cléistogames conformes à l'invention peut conférer en outre les avantages suivants :
- l'amélioration du rendement, du fait d'une réflexion plus faible du rayonnement incident lors de la floraison du colza ;
- la limitation des risques de développement de *Sclerotinia sclerotiorum* (champignon parasite du colza), du fait que la proportion de pétales restant collés sur les feuilles est beaucoup plus faible chez les mutants conformes à l'invention que chez les colzas habituellement utilisés.

Enfin, la cléistogamie confère à la fleur une morphologie originale, qui peut être utilisée pour l'obtention de plantes ornementales.

Les différentes applications mentionnées ci-dessus peuvent également être envisagées dans le cas d'autres crucifères, en particulier le chou et navette, progéniteurs du colza.

La présente invention sera mieux comprise à l'aide du complément de description qui va suivre, qui se réfère à des exemples non-limitatifs d'obtention et de caractérisation d'un mutant de colza conforme à l'invention.

### EXEMPLE 1: INDUCTION DE LA MUTATION

Des graines Mo de la variété de colza d'hiver sans acide érucique "Primor" ont été traitées avec un agent mutagène, le MSE, selon le protocole suivant:
- concentration : 0,5% de MSE en solution aqueuse ;
- traitement pendant 16 heures sur un agitateur rotatif ;
- rinçage 3 fois avec de l'eau ;
- Séchage avant semis direct au champ des graines M1.

Un mutant cléistogame a été mis en évidence à la floraison dans la descendance M2. Ce mutant, dénommé J323 a été fixé à l'état homozygote sur la génération M3.

La mutation obtenue a été nommée "C1g1".

### EXEMPLE 2 : CARACTERISATION DU MUTANT

### 1- Caractérisation morphologique :

Le mutant J323 présente des fleurs complètes mais qui ne s'ouvrent pas à l'anthèse ; après un développement normal, les fleurs restent en général fermées jusqu'à la chute des pétales, qui a lieu après la fécondation.

Les étamines et le stigmate restent ainsi enfermés dans le calice. Il apparaît que seul ce caractère est affecté par la mutation. Les autres organes de la fleur (les 4 sépales, les 4 nectaires, le style et le stigmate, ainsi que les 6 étamines) se développent normalement.

Ce mutant peut être considéré comme autogame strict.

Ce caractère floral a des conséquences directes sur la dispersion du pollen:
- par le vent, la fleur étant fermée, les étamines sont pour ainsi dire ensachées
- par les insectes, la plupart des abeilles collectant le nectar en visitant la fleur en position insérante et non surmontante sans passer sur les étamines ou le stigmate de la fleur donc sans participer à la pollinisation de la fleur.

### 2-Caractérisation génétique du caractère cléistogame

Après avoir été fixée, la lignée obtenue à partir du mutant cléistogame 'J323' a été croisée avec deux lignées de colza d'hiver double zéro et à reproduction mixte : "Darmor" et "Samouraï".

Les plantes F1 ainsi obtenues présentent des fleurs partiellement ouvertes traduisant le caractère additif de la mutation.

Ces plantes F1 ont été autofécondées pour produire la descendance F2 nécessaire à l'étude du déterminisme génétique du caractère cléistogame. Les résultats sont résumés ci-dessous:

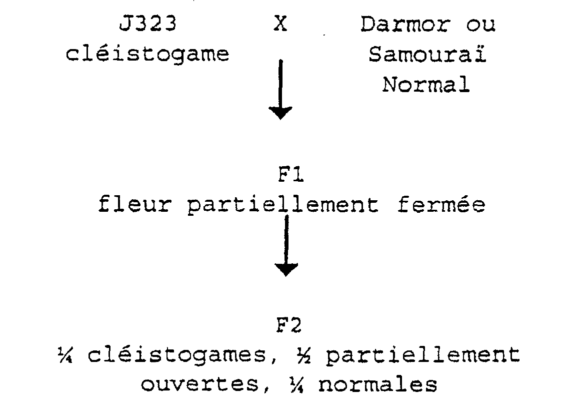

En F2, la proportion de plantes cléistogames est d'environ 25%:
- Darmor: 246/1000 = 24.6% ;
- Samouraï: 49/222 = 22.1 %.

Ces résultats montrent que le caractère cléistogame est contrôlé par un seul gène, qui a été appelé "*clg1*".

Il a ensuite été transféré dans la descendance en rétrocroisement (B1Fn) avec les deux lignées "Darmor" et "Samouraï".

Ce caractère peut donc être transféré dans des lignées ou des populations par sélection généalogique, haploïdisation ou rétrocroisement.

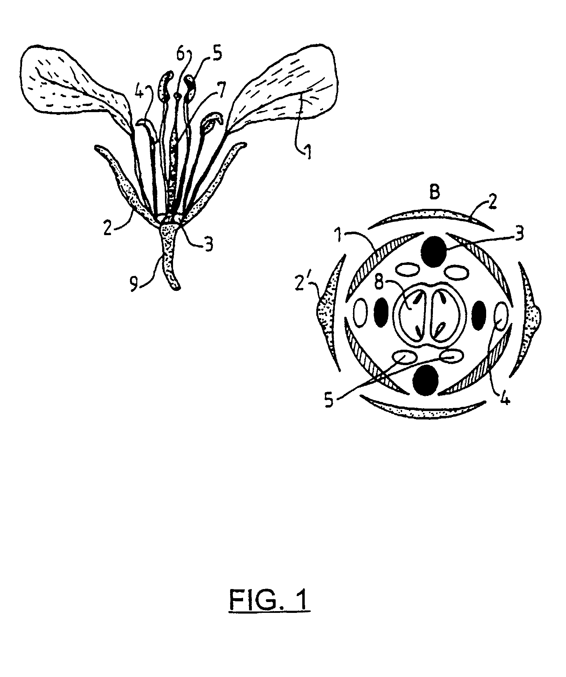

## Revendications

1. Procédé d'obtention d'un mutant cléistogame de crucifère, **caractérisé en ce qu'**il comprend au moins les étapes suivantes :
a) la mutation d'au moins une cellule de crucifère ;
b) l'obtention, à partir de ladite cellule, d'une plante adulte présentant à maturité florale des fleurs totalement closes.

2. Procédé selon la revendication 1 **caractérisé en ce que** l'étape b) comprend les sous-étapes suivantes :
c) l'obtention de plantes adultes présentant à maturité florale des fleurs incomplètement ouvertes ;
d) le croisement desdites plantes entre elles, et la sélection parmi leurs descendants, d'au moins une plante adulte présentant à maturité florale des fleurs totalement closes.

3. Procédé selon une quelconque des revendications 1 ou 2, **caractérisé en ce que** la mutation de l'étape a) est effectuée en traitant des semences de crucifère par un agent mutagène.

4. Procédé selon la revendication 3, **caractérisé en ce que** ledit agent mutagène est l'éthyl-méthyl sulfonate.

5. Procédé selon une quelconque des revendications 1 à 4, **caractérisé en ce que** ladite crucifère est choisie dans le groupe constitué par le colza, le chou, la navette, et la moutarde.

6. Mutant cléistogame de crucifère, susceptible d'être obtenu par le procédé selon une quelconque des revendications 1 à 5.

7. Utilisation d'un mutant cléistogame de crucifère, selon la revendication 6, pour la reproduction autogame de ladite crucifère.

## Patentansprüche

1. Verfahren zur Herstellung einer kleistogamen Kreuzblütler-Mutante,
**dadurch gekennzeichnet,**
**dass** es mindestens die folgenden Stufen umfasst:
a) Mutation mindestens einer Kreuzblütler-Zelle;
b) ausgehend von der Zelle Herstellung einer ausgewachsenen Pflanze, die in der Blühreife vollständig geschlossene Blüten aufweist.

2. Verfahren nach Anspruch 1,
**dadurch gekennzeichnet,**
**dass** die Stufe b) die folgenden Unterstufen umfasst:
c) Herstellung von ausgewachsenen Pflanzen, die in der Blühreife unvollständig geöffnete Blüten aufweisen;
d) Kreuzung der genannten Pflanzen untereinander und Selektion mindestens einer ausgewachsenen Pflanze unter den Abkömmlingen, welche in der Blühreife vollständig geschlossene Blüten aufweist.

3. Verfahren nach Anspruch 1 oder Anspruch 2, **dadurch gekennzeichnet,**
**dass** die Mutation der Stufe a) durch Behandlung der Kreuzblütler-Samen mit einem mutagenen Agens durchgeführt wird.

4. Verfahren nach Anspruch 3,
**dadurch gekennzeichnet,**
**dass** das mutagene Agens Ethylmethylsulfonat ist.

5. Verfahren nach einem der Ansprüche 1 bis 4,
**dadurch gekennzeichnet,**
**dass** der Kreuzblütler aus der Gruppe bestehend aus Raps, Kohl, Rüben und Senf ausgewählt wird.

6. Kleistogame Kreuzblütler-Mutante, die durch das Verfahren nach einem der Ansprüche 1 bis 5 erhalten werden kann.

7. Verwendung einer kleistogamen Kreuzblütler-Mutante nach Anspruch 6 zur autogamen Fortpflanzung des Kreuzblütlers.

## Claims

1. A process for producing a cleistogamous mutant of a crucifer, **characterised in that** it comprises at least the following steps:
a) the mutation of at least one crucifer cell;
b) the production, from said cell, of an adult plant exhibiting totally closed flowers at floral maturity.

2. The process as claimed in claim 1, **characterised in that** step b) comprises the following substeps:
c) the production of adult plants exhibiting incompletely open flowers at floral maturity;
d) the mutual crossing of said plants and the selection, from their progeny, of at least one adult plant exhibiting totally closed flowers at floral maturity.

3. The process as claimed in either of claims 1 or 2, **characterised in that** the mutation in step a) is carried out by treating crucifer seeds with a mutagenic agent.

4. The process as claimed in claim 3, **characterised in that** said mutagenic agent is ethyl methyl sulfonate.

5. The process as claimed in any one of claims 1 to 4, **characterised in that** said crucifer is chosen from the group consisting of rapeseed, cabbage, naven and mustard.

6. A cleistogamous mutant of a crucifer which is capable of being produced by the process as claimed in any one of claims 1 to 5.

7. The use of a cleistogamous mutant of a crucifer as claimed in claim 6 for the autogamous reproduction of said crucifer.
